# EUROPEAN PATENT APPLICATION

(11) **EP 4 653 000 A1**
(43) Date of publication of application: **26.11.2025**
(21) Application number: 24178040.2
(22) Date of filing: 24.05.2024
(51) Int. Cl.: A61K 31/662, A61K 45/06, A61P 27/02, A61P 31/04

(54) **INHIBITORS OF PSEUDOMONAS AERUGINOSA ELASTASE (LASB) FOR THE TREATMENT OF PSEUDOMONAS KERATITIS**

(71) Applicant: Helmholtz-Zentrum für Infektionsforschung GmbH, 38124 Braunschweig (DE); Universität des Saarlandes, 66123 Saarbrücken (DE)
(72) Inventor: HIRSCH, ANNA, 38124 Braunschweig (DE); HAUPENTHAL, JÖRG, 38124 Braunschweig (DE); SCHÜTZ, CHRISTIAN, 38124 Braunschweig (DE); KANY, ANDREAS, 38124 Braunschweig (DE); SHAFIEI, ROYA, 38124 Braunschweig (DE); SPEICHER, SAMIRA, 38124 Braunschweig (DE); ENGLISCH, COLYA, 66123 Saarbrücken (DE); BISCHOFF, MARKUS, 66123 Saarbrücken (DE); WADOOD, NORAN A., 66123 Saarbrücken (DE)
(74) Representative: Forstmeyer, Dietmar

(57) **Abstract**

The present invention relates to the use of inhibitors of the *Pseudomonas aeruginosa* virulence factor LasB for the treatment of *Pseudomonas* Keratitis.

## Description

The present invention relates to the use of inhibitors of the *Pseudomonas aeruginosa* virulence factor LasB for the treatment of *Pseudomonas* Keratitis.

*P. aeruginosa* is a Gram-negative bacterium, which is ranked by the WHO as one of the most critical pathogens today (World Health Organization. Global Priority List of Antibiotic-Resistant Bacteria to Guide Research, Discovery, and Development of New Antibiotics. WHO 2017). This opportunistic bacterium causes around 10% of hospital-acquired infections and has a high occurrence among immunocompromised and cystic-fibrosis patients (Magill, S. S.; Edwards, J. R.; Bamberg, W.; Beldavs, Z. G.; Dumyati, G.; Kainer, M. A.; Lynfield, R.; Maloney, M.; McAllister-Hollod, L.; Nadle, J.; et al. N. Engl. J. Med. 2014, 370, 1198-1208; Richards, M. J.; Edwards, J. R.; Culver, D. H.; Gaynes, R. P. Pediatrics 1999, 103, e39; Valenza, G.; Tappe, D.; Turnwald, D.; Frosch, M.; König, C.; Hebestreit, H.; Abele-Horn, M. J. Cyst. Fibros. 2008, 7, 123-127; Sorde, R.; Pahissa, A.; Rello, J. Infect. Drug Resist. 2011, 4, 31-41). The development of potent antibiotics is urgently needed due to the lack of efficient therapeutics on the market (Mesaros, N.; Nordmann, P.; Plésiat, P.; Roussel-Delvallez, M.; Eldere, J. Van; Glupczynski, Y.; Laethem, Y. Van; Jacobs, F.; Lebecque, P.; Malfroot, A.; et al. Clin. Microbiol. Infect. 2007, 13, 560-578; Taubes, G. Science 2008, 321, 356-361). This task is complicated by the high intrinsic resistance of the pathogen (Hancock, R. E. W.; Speert, D. P. Drug Resist. Updat. 2000, 3, 247-255; Strateva, T.; Yordanov, D. J. Med. Microbiol. 2009, 58, 1133-1148).

Keratitis is an inflammation of the cornea, characterized by rapidly advancing destruction of the stroma that can lead to complications like thinning or perforation of the cornea, but also to blindness (doi: 10.4103/meajo.MEAJO_264_16; 10.1016/j.micpath.2016.12.013). Consequently, the main unmet medical need is the liquefactive necrosis of the cornea with major stromal melting and perforation in *Pseudomonas* keratitis, and in particular, in contact lens-associated keratitis (CLAK). Epidemiology indicates more than two million cases of microbial keratitis yearly (doi: 10.1016/j.survophthal.2018.12.003), with *P. aeruginosa* being one of the major causal agents, especially among contact lens wearers (DOI 10.1186/s12886-017-0612-2). Besides being a health threatening condition, it is also an economic burden (doi: 10.1038/s41433-020-01360-6). Current solutions to treat *P*. *aeruginosa* keratitis rely on the treatment with classical antibiotics, such as chloramphenicol, gentamicin, kanamycin, levofloxacin and ofloxacin.

A new approach in the field of the development of anti-infectives is the anti-virulence strategy, which also applies to LasB. The aim of this strategy is not to kill the bacteria, but to reduce their pathogenicity, thereby reducing selection pressure. This approach e.g. has the advantage that it is effective even if pseudomonads that are resistant to standard-of-care (SOC) antibiotics need to be treated; or by inhibiting the extracellular LasB even after the bacteria have died. Several LasB inhibitors are disclosed in WO 2023/166039 A1 and WO 2022/043322 A1 and documents cited therein.

The role of *P. aeruginosa* elastase (LasB) - which is able to hydrolyse a vast array of host proteins, causing damage to tissues, disrupting host immune responses, and promoting inflammation (doi: 10.1016/j.drudis.2021.02.026) - in keratitis has been discussed in the literature. Thereby, Ohman et al (doi: 10.1093/infdis/142.4.547), Preston et al (doi: 10.1128/iai.65.8.3086-3090.1997) and Hobden et al (doi: 10.1089/10445490260099674) concluded, that LasB does not contribute to pathogenicity in corneal infections in mice. This also contradicts older publications from 1978 to 1990 in which eyes were treated (*in vivo*) with extracellular proteases of *P*. *aeruginosa* (doi: 10.1128/iai.19.2.630-648.1978; Kreger and Gray, 1978), LasB enzyme (PMID: 6409834; Kessler et al, 1983) or a *P*. *aeruginosa* strain called PA-28 (doi: 10.1128/AAC.34.11.2065; Burns et al., 1990) - where a relevant role of LasB in the course of the disease is suspected. However, these trials suffer from inconsistencies such as the use of a protease mixture (Kreger and Gray), questionable conclusions based on the treatment with peptidic inhibitors like phosphoramidon (Kessler), which is a "notoriously promiscuous zinc endopeptidase inhibitor, inhibiting human endothelin-converting enzyme, neutral endopeptidases, and MMPs, among others" (comment in doi: 10.1016/j.drudis.2021 .02.026, Everett and Davies, 2021). As a result, unspecific effects may have occurred due to these off-target activities. To conclude, particularly the above-mentioned publications by Ohman and the more recent publications by Preston as well as Hobden strongly suggest that LasB is not a suitable target for the treatment of Pseudomonas keratitis.

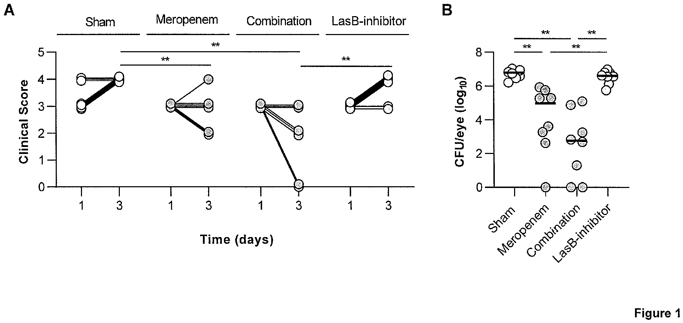

The inventors of the present invention have however surprisingly found that LasB also plays an important role in this disease process, especially in the early and acute phase of infection, and that inhibition of this protease has a favorable effect on the progression of the disease (see Figures 1-4). The contradiction to the findings by other groups can possibly be explained by the fact that for the first time experiments were conducted using a non-toxic, highly selective small molecule with IC₅₀ in the single-digit nanomolar range to treat an eye infection with a relevant clinical isolate of *P*. *aeruginosa,* which has been proven to express LasB (quantified using qPCR). Accordingly, the present invention provides inhibitors of the *P. aeruginosa* virulence factor LasB for use in the treatment of *Pseudomonas* Keratitis.

The present invention provides compounds of formula (I) 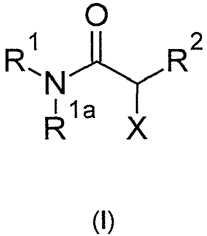 wherein
X is a group of formula -PO(OH)₂, -SH, -C(=O)-NH-OH, an optionally substituted triazolyl group, -SR³, -PO(OH)(OR⁴) or -PO(OR⁴)(OR⁵);
R¹ is an optionally substituted cycloalkyl group, an optionally substituted heterocycloalkyl group, an optionally substituted aryl group or an optionally substituted heteroaryl group or an optionally substituted aralkyl group or an optionally substituted heteroaralkyl group; or a group of formula -CH(R⁶)-C(=O)-NH-R⁷, or a group of formula -C(Me)₂-CH₂-C(=O)-NH-R⁷, or a group of formula -CH(R⁶)-CH₂-C(=O)-NH-R⁷, or a group of formula -CH(R⁶)-R⁸;
R² is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R³ is a group of formula -COR^{3a} or -CON(R^{3b})₂; wherein R^{3a} is an alkyl group, an optionally substituted phenyl group or an optionally substituted benzyl group and R^{3b} is independently selected from hydrogen or an alkyl group, an optionally substituted phenyl group or an optionally substituted benzyl group;
R⁴ is an alkyl group, an optionally substituted phenyl group or an optionally substituted benzyl group;
R⁵ is an alkyl group, an optionally substituted phenyl group or an optionally substituted benzyl group;
R⁶ is hydrogen or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R⁷ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group;
R⁸ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group; and
R^{1a} is hydrogen, or, if R¹ is a group of formula -CH(R⁶)-C(=O)-NH-R⁷, R^{1a} and R⁶ together may be a group of formula -(CH₂)₃- or -(CH₂)₄-;
or a salt thereof;
for use in the treatment of *Pseudomonas* Keratitis.

The present invention further provides compounds of formula (Ia) 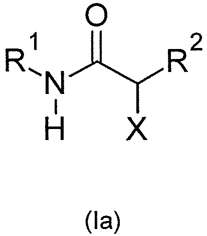 wherein
X is a group of formula -PO(OH)₂, -SH, -C(=O)-NH-OH, an optionally substituted triazolyl group, -SR³, -PO(OH)(OR⁴) or -PO(OR⁴)(OR⁵);
R¹ is an optionally substituted cycloalkyl group, an optionally substituted heterocycloalkyl group, an optionally substituted aryl group or an optionally substituted heteroaryl group or an optionally substituted aralkyl group or an optionally substituted heteroaralkyl group; or a group of formula -CH(R⁶)-C(=O)-NH-R⁷, or a group of formula -C(Me)₂-CH₂-C(=O)-NH-R⁷, or a group of formula -CH(R⁶)-CH₂-C(=O)-NH-R⁷, or a group of formula -CH(R⁶)-R⁸;
R² is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R³ is a group of formula -COR^{3a} or -CON(R^{3b})₂; wherein R^{3a} is an alkyl group, an optionally substituted phenyl group or an optionally substituted benzyl group and R^{3b} is independently selected from hydrogen or an alkyl group, an optionally substituted phenyl group or an optionally substituted benzyl group;
R⁴ is an alkyl group, an optionally substituted phenyl group or an optionally substituted benzyl group;
R⁵ is an alkyl group, an optionally substituted phenyl group or an optionally substituted benzyl group;
R⁶ is hydrogen or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R⁷ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group; and
R⁸ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group;
or a salt thereof;
for use in the treatment of *Pseudomonas* Keratitis.

Preferably, X is a group of formula -PO(OH)₂, -SH, -C(=O)-NH-OH or a triazolyl group.

The present invention moreover provides compounds of formula (Ia) 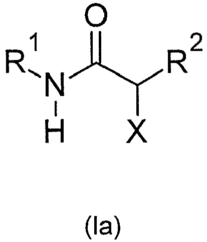 wherein
X is a group of formula -SH, -PO(OH)₂, -SR³, -PO(OH)(OR⁴) or -PO(OR⁴)(OR⁵);
R¹ is an optionally substituted aryl group or an optionally substituted heteroaryl group;
R² is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R³ is a group of formula -COR^{3a} or -CON(R^{3b})₂; wherein R^{3a} is an alkyl group, an optionally substituted phenyl group or an optionally substituted benzyl group and R^{3b} is independently selected from hydrogen or an alkyl group, an optionally substituted phenyl group or an optionally substituted benzyl group;
R⁴ is an alkyl group, an optionally substituted phenyl group or an optionally substituted benzyl group;
R⁵ is an alkyl group, an optionally substituted phenyl group or an optionally substituted benzyl group;
or a salt thereof;
for use in the treatment of *Pseudomonas* Keratitis.

According to a further preferred embodiment, the present invention provides compounds of formula (II) 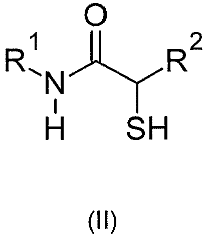 wherein R¹ and R² are as defined above or below; or a salt thereof; for use in the treatment of *Pseudomonas* Keratitis.

According to a moreover preferred embodiment, the present invention provides compounds of formula (II) 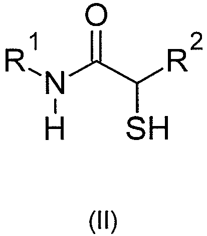 wherein
R¹ is an optionally substituted aryl group or an optionally substituted heteroaryl group;
   and
R² is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
or a salt thereof;
for use in the treatment of *Pseudomonas* Keratitis.

According to a further preferred embodiment, the present invention provides compounds of formula (III) 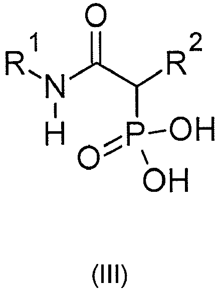 wherein R¹ and R² are as defined above or below; or a salt thereof; for use in the treatment of *Pseudomonas* Keratitis.

According to a moreover preferred embodiment, the present invention provides compounds of formula (III) 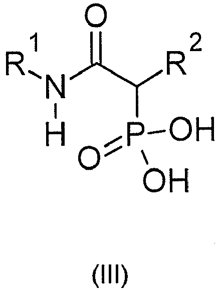 wherein
R¹ is an optionally substituted aryl group or an optionally substituted heteroaryl group;
   and
R² is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
or a salt thereof;
for use in the treatment of *Pseudomonas* Keratitis.

According to a further preferred embodiment, the present invention provides compounds of formula (IV) 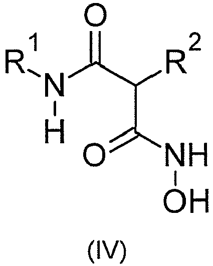 wherein R¹ and R² are as defined above or below; or a salt thereof; for use in the treatment of *Pseudomonas* Keratitis.

According to a moreover preferred embodiment, the present invention provides compounds of formula (V) 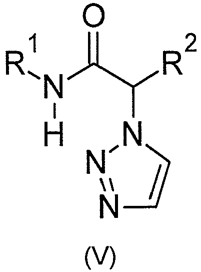 wherein R¹ and R² are as defined above or below; or a salt thereof; for use in the treatment of *Pseudomonas* Keratitis.

According to a further preferred embodiment, the present invention provides compounds of formula (VI) 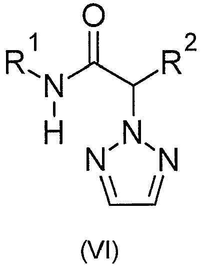 wherein R¹ and R² are as defined above or below; or a salt thereof; for use in the treatment of *Pseudomonas* Keratitis.

The following preferred embodiments independently apply to compounds of formulas (I), (Ia), (II), (III), (IV), (V) and (VI):
Preferably, R¹ is an optionally substituted cycloalkyl group, an optionally substituted heterocycloalkyl group, an optionally substituted aryl group or an optionally substituted heteroaryl group or an optionally substituted aralkyl group or an optionally substituted heteroaralkyl group.

Further preferably, R¹ is an optionally substituted aryl group or an optionally substituted heteroaryl group.

Moreover preferably, R¹ is an optionally substituted phenyl group, an optionally substituted naphthyl group or an optionally substituted heteroaryl group containing one or two rings and from 5 to 10 ring atoms selected from C, O, N and S.

Especially preferably, R¹ is an optionally substituted phenyl group or an optionally substituted heteroaryl group containing one or two rings and 5, 6, 9 or 10 ring atoms selected from C, O, N and S.

Further preferably, R¹ is an optionally substituted heteroaryl group containing 5 or 6 ring atoms selected from C, O, N and S.

More preferably, R¹ is an optionally substituted phenyl group.

Further preferably, R¹ is a group of formula -Cy¹-L-Cy², wherein Cy¹ is an optionally substituted cycloalkylene group containing 1 or 2 rings and from 3 to 7 carbon ring atoms, an optionally substituted heterocycloalkylene group containing 1 or 2 rings and from 3 to 7 ring atoms selected from C, N, O and S, an optionally substituted phenylene group, or an optionally substituted heteroarylene group containing 5 or 6 ring atoms selected from C, N, O and S; Cy² is a cycloalkyl, heterocycloalkyl, alkylcycloalkyl, heteroalkylcycloalkyl, aryl, heteroaryl, aralkyl or heteroaralkyl group, all of which may optionally be substituted; and L is a bond or -O-, -S-, -NH-, -CH₂-, -CO-, -NHCO-, -CO-NH-, -CH₂-CO-NH-, -NH-CO-CH₂-, -CH₂-O-CO-NH-, -NH-CO-O-CH₂-, -O-CO-NH-, - NH-CO-O-, -NHSO₂-, -SO₂NH-, -CH₂-SO₂-NH-, -NH-SO₂-CH₂-, -S-CH₂-, -CH₂-S-, - NH-CH₂-, -CH₂-NH-, -O-CH₂- or -CH₂-O-.

Preferably, Cy² is an optionally substituted phenyl group, an optionally substituted biphenyl group, an optionally substituted naphthyl group, an optionally substituted heteroaryl group containing one or two rings and 5, 6, 9 or 10 ring atoms selected from C, O, N and S, an optionally substituted cycloalkyl group containing from 3 to 7 ring atoms, an optionally substituted heterocycloalkyl group containing from 3 to 7 ring atoms selected from C, N, O and S, an optionally substituted heterocycloalkylaryl group containing 9 or 10 ring atoms selected from C, N, S and O, or a group of formula -CH(CH₂Ph)Ph.

Further preferably, L is a bond or -NHCO-, -CO-NH-, -CH₂-CO-NH-, -NH-CO-CH₂-, - NHSO₂- or -SO₂NH-.

Moreover preferably, Cy¹ is a 1 ,4-phenylene group.

Further preferably, R¹ is a group of formula -CH(R⁶)-C(=O)-NH-R⁷.

Moreover preferably, R¹ is a group of formula -CH(R⁶)-R⁸.

Further preferably, R⁶ is hydrogen or a C₁₋₆ alkyl group, a C₃₋₇ cycloalkyl group, a heterocycloalkyl group containing from 3 to 7 ring atoms selected from C, N, O and S, a phenyl group or a heteroaryl group containing 5 or 6 ring atoms selected from C, N, S and O, or a group of formula -CH₂-R^{6a}, wherein R^{6a} is a C₃₋₇ cycloalkyl group, a heterocycloalkyl group containing from 3 to 7 ring atoms selected from C, N, O and S, a phenyl group or a heteroaryl group containing 5 or 6 ring atoms selected from C, N, S and O.

Especially preferably, R⁶ is a -CH(CH₃)₂ group.

Moreover preferably, R⁷ is an optionally substituted phenyl group or an optionally substituted C₃₋₇ cycloalkyl group; especially an optionally substituted phenyl group.

Further preferably, R⁸ is an optionally substituted heteroaryl group containing 5 to 10 ring atoms selected from C, N, O and S.

Further preferably, R⁸ is an optionally substituted benzimidazole group or an optionally substituted triazole group or an optionally substituted imidazole group.

Moreover preferably, R⁸ is a group of the following formula: 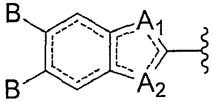 wherein each ".....", independently of one another, represents a single bond or a double bond, wherein at least one "....." in each of the rings is a double bond;
A₁ and A₂ each, independently of one another represents CH, N, NH, O or S;
B is R^{B1} or a group of formula -Y-R^{B2}, wherein
R^{B1} is a hydrogen atom, a halogen atom, CN, CF₃, CH₂-OH; NR^{T1}R^{T2}; or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted;
R^{T1} and R^{T2} each, independently of one another, represents a hydrogen atom or a (C₁-C₃) alkyl group, which may be substituted by one or more, identical or different, group(s) selected from a halogen atom, OH, =O, and NH₂;
Y is -O- or -S-; and
R^{B2} is a hydrogen atom or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which groups may optionally be substituted.

Further preferably, R⁸ is a group of the following formula: 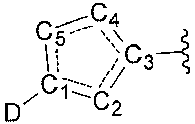 wherein each ".....", independently of one another, represents a single bond or a double bond, wherein at least one " " is a double bond;
C₁ and C₃ each, independently of one another represents C or N;
C₂, C₄ and C₅ each, independently of one another represents CH, N, NH, O, or S;
D is an optionally substituted aryl group or an optionally substituted heteroaryl group (especially preferably, D is an optionally substituted phenyl group).

Further preferably, R⁸ is a group of the following formula: 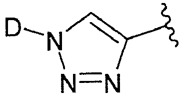 wherein D is an optionally substituted aryl group or an optionally substituted heteroaryl group (especially preferably, D is an optionally substituted phenyl group).

Further preferably, R² is a C₁₋₆ alkyl group; a heteroalkyl group containing from 1 to 6 carbon atoms and 1, 2, 3 or 4 heteroatoms selected from O, S and N; a C₄₋₁₀ alkylcycloalkyl group; or a C₇₋₁₂ aralkyl group; all of which may optionally be substituted.

Especially preferably, R² is a C₁₋₆ alkyl group; a heteroalkyl group containing from 1 to 6 carbon atoms and 1, 2, 3 or 4 heteroatoms selected from O, S and N; or a group of formula -CH₂-R²¹, wherein R²¹ is a C₃₋₇ cycloalkyl group, COOH, COOMe or an optionally substituted phenyl group.

Moreover, especially preferably, R² is a C₁₋₄ alkyl group; or a group of formula -CH₂-R²¹, wherein R²¹ is a C₃₋₆ cycloalkyl group, OMe, COOH, COOMe or an optionally substituted phenyl group. Preferably, R²¹ is a phenyl group which is unsubstituted or substituted by one or two substituents which are independently selected from OH, NO₂ and Me; further preferably, R²¹ is an unsubstituted phenyl group.

More preferably, R² is an optionally substituted benzyl group (i.e., a group of formula -CH₂-Ph which may optionally be substituted). Moreover preferably, R² is an unsubstituted benzyl group.

Further preferably, R² is an iso-butyl group (i.e., a group of formula -CH₂CH(CH₃)₂).

The present invention moreover provides compounds of formula (VII) 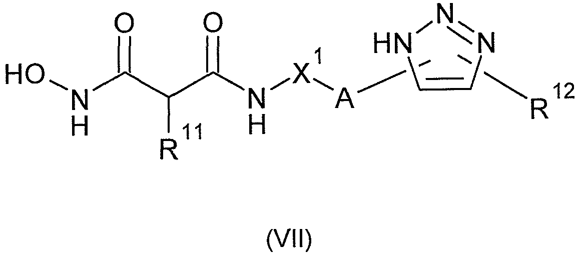 wherein
A is a bond, CH₂ or C=O;
X¹ is an optionally substituted cycloalkylene group, an optionally substituted heterocycloalkylene group, an optionally substituted arylene group or an optionally substituted heteroarylene group;
R¹¹ is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted; and
R¹² is hydrogen or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
or a salt thereof;
for use in the treatment of *Pseudomonas* Keratitis.

Preferably, A is CH₂.

The present invention moreover provides compounds of formula (VIIa) 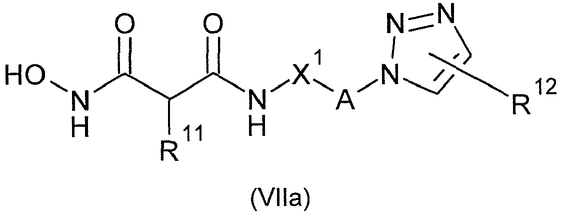 wherein A, X¹, R¹¹ and R¹² are as defined above or below, or a salt thereof; for use in the treatment of *Pseudomonas* Keratitis.

The present invention further provides compounds of formula (VIII) 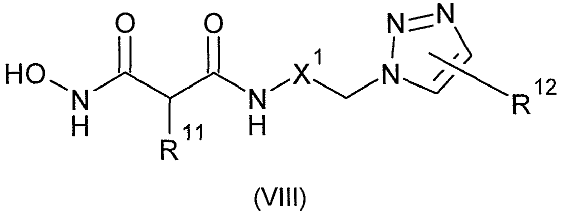 wherein X¹, R¹¹ and R¹² are as defined above or below, or a salt thereof; for use in the treatment of *Pseudomonas* Keratitis.

Preferably, X¹ is an optionally substituted arylene group or an optionally substituted heteroarylene group;

Further preferably, X¹ is an optionally substituted phenylene group or an optionally substituted heteroarylene group having 5 or 6 ring atoms that are selected from C, N, O and S.

Especially preferably, X¹ is a 1,3 phenylene group.

Further especially preferably, X¹ is a 1,4 phenylene group.

Further preferably, R¹¹ is a C₁₋₆ alkyl group; a heteroalkyl group containing from 1 to 6 carbon atoms and 1, 2, 3 or 4 heteroatoms selected from O, S and N; a C₃₋₇ cycloalklyl group; a C₄₋₁₀ alkylcycloalkyl group; or a C₇₋₁₂ aralkyl group; all of which may optionally be substituted.

Moreover preferably, R¹¹ is a C₁₋₆ alkyl group; a heteroalkyl group containing from 1 to 6 carbon atoms and 1, 2, 3 or 4 heteroatoms selected from O, S and N; a C₃₋₇ cycloalklyl group; or a group of formula -CH₂-R^{11a}, wherein R^{11a} is a C₃₋₇ cycloalkyl group or an optionally substituted phenyl group.

Further preferably, R¹¹ is a C₁₋₆ alkyl group; or a group of formula -CH₂-R^{11a}, wherein R^{11a} is a phenyl group or a cyclopropyl group.

Further preferably, R¹¹ is a C₁₋₄ alkyl group; or a group of formula -CH₂-R^{11a}, wherein R^{11a} is a phenyl group or a cyclopropyl group.

Especially preferably, R¹¹ is an *iso*-butyl group (i.e., a group of formula -CH₂CH(CH₃)₂).

Moreover preferably, R¹¹ is an *iso*-propyl group (i.e., a group of formula -CH(CH₃)₂).

Further preferably, R¹² is a group of formula -CH₂-NH-SO₂-R¹³ or -CH₂-N(CH₃)-SO₂-R¹³, wherein R¹³ is an optionally substituted phenyl group, an optionally substituted benzyl group, an optionally substituted C₃₋₁₀ cycloalkyl group, an optionally substituted -CH₂-C₃₋₁₀ cycloalkyl group, a C₁₋₆ alkyl group or a C₁₋₆ heteroalkyl group.

Moreover preferably, R¹² is a group of formula -CH₂-Y¹-R¹⁴, wherein Y¹ is selected from a bond, O, NH, S and NHCO; and R¹⁴ is hydrogen, a C₁₋₆ heteroalkyl group or an optionally substituted phenyl group (preferably, R¹⁴ is an optionally substituted phenyl group).

Further preferably, R¹² is an optionally substituted phenyl group.

Preferably, the optional substituents (preferably, 1 or 2 substituent(s)) at group R¹², R¹³ or R¹⁴ are independently selected from halogen atoms (e.g., F, Cl, Br, I), OH, NH₂, COOH, =O, phenyl, C₁₋₆ alkyl groups and C₁₋₆ heteroalkyl groups.

Moreover preferably, R¹² is selected from the following groups:

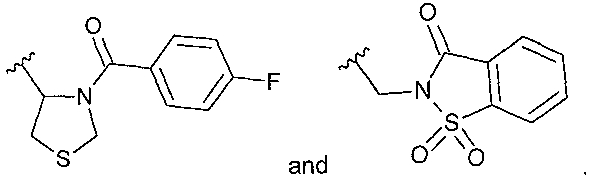

The most preferred compounds of the present invention are the compounds disclosed in the examples, or a salt thereof.

Especially preferred is the following compound, or a salt thereof:

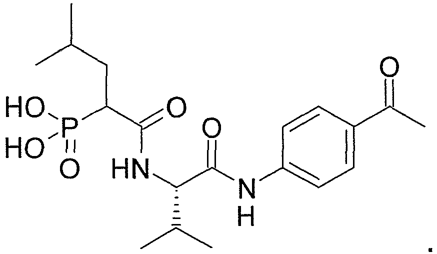

It is further preferred to combine the preferred embodiments of the present invention in any desired manner (e.g., any embodiment for R¹ may be combined with any embodiment of R²).

The term "optionally substituted" refers to a group which is unsubstituted or substituted by one or more (especially by one, two or three; preferably by one or two) substituents.

If a group (e.g., group R¹ and/or group R²) comprises more than one substituent, these substituents are independently selected, i.e., they may be the same or different.

If a group (e.g., group R¹ and/or group R²) is substituted by a cyclic group, such as e.g., a cycloalkyl group or a heterocycloalkyl group, this cyclic group may be bonded to this group (e.g., group R¹ and/or group R²) via a single or double bond or this cyclic group may be annulated or fused to said group (e.g., group R¹ and/or group R²). Isatin is an example for a substituted phenyl group.

Examples for substituents are fluorine, chlorine, bromine and iodine and OH, =O, SH, NH₂, -SO₃H, -SO₂NH₂, -COOH, -COOMe, -COMe (Ac), -NHSO₂Me, -SO₂NMe₂, - CH₂NH₂, -NHAc, -SO₂Me, -CONH₂, -CN, -NHCONH₂, -NHC(NH)NH₂, -NOHCH₃, -N₃ and -NO₂ groups. Further examples of substituents are C₁-C₁₀ alkyl, C₂-C₁₀ alkenyl, C₂-C₁₀ alkynyl, C₁-C₁₀ heteroalkyl, C₃-C₁₈ cycloalkyl, C₁-C₁₇ heterocycloalkyl, C₄-C₂₀ alkylcycloalkyl, C₁-C₁₉ heteroalkylcycloalkyl, C₆-C₁₈ aryl, C₁-C₁₇ heteroaryl, C₇-C₂₀ aralkyl and C₁-C₁₉ heteroaralkyl groups; especially C₁-C₆ alkyl, C₂-C₆ alkenyl, C₂-C₆ alkynyl, C₁-C₆ heteroalkyl, C₃-C₁₀ cycloalkyl, C₁-C₉ heterocycloalkyl, C₄-C₁₂ alkylcycloalkyl, C₁-C₁₁ heteroalkylcycloalkyl, C₆-C₁₀ aryl, C₁-C₉ heteroaryl, C₇-C₁₂ aralkyl and C₁-C₁₁ heteroaralkyl groups, further preferably C₁-C₆ alkyl and C₁-C₆ heteroalkyl groups.

Preferred substituents are halogen atoms (e.g., F, Cl, Br, I) and groups of formula - OH, =O, -O-C₁₋₆ alkyl (e.g., -OMe, -OEt, -O-*n*Pr, -O-*i*Pr, -O-*n*Bu, -O-*i*Bu and -O-*t*Bu), - NH₂, -NHC₁₋₆ alkyl, -N(C₁₋₆ alkyl)₂, -COOH, -COO-C₁₋₆ alkyl (e.g., -COOMe), -CO-C₁₋₆ alkyl (e.g., -COMe), -COCF₃, -NHSO₂Me, -SO₂NMe₂, -OCH₂CH₂OCH₃, -SO₃H, - SO₂NH₂, -CONH₂, -CH₂NH₂, -CN, -C₁₋₆ alkyl (e.g., -Me, -Et, -*n*Pr, -*i*Pr, -*n*Bu, -*i*Bu, -*t*Bu and -CF₃), -C₁₋₆ heteroalkyl, -SH, -S-CO-C₁₋₆ alkyl, -S-C₁₋₆ alkyl, -NHAc, -NO₂, -C≡CH, -NHCONH₂, -SO₂Me, -SO₂CF₃, phenyl, -CO-4-fluorophenyl, -C₃₋₆ cycloalkyl (e.g. cyclopropyl, cyclobutyl) and heterocycloalkyl containing 3 to 6 ring atoms selected from C, N, S and O.

Moreover preferred substituents are halogen atoms (e.g., F, Cl, Br, I), OH, NH₂, COOH, =O, phenyl, C₁₋₆ alkyl groups and C₁₋₆ heteroalkyl groups.

Further preferred substituents are halogen atoms (e.g., F, Cl, Br, I) and groups of formula -OH, =O, -O-C₁₋₆ alkyl (e.g., -OMe, -OEt, -O-*n*Pr, -O-*i*Pr, -O-*n*Bu, -O-*i*Bu and - O-*t*Bu), -NH₂, -NHC₁₋₆ alkyl, -N(C₁₋₆ alkyl)₂, -COOH, -COO-C₁₋₆ alkyl (e.g., -COOMe), - CO-C₁₋₆ alkyl (e.g., -COMe), -OCH₂CH₂OCH₃, -NHSO₂Me, -SO₂NMe₂, -SO₃H, - SO₂NH₂, -CONH₂, -CH₂NH₂, -CN, -C₁₋₆ alkyl (e.g., -Me, -Et, -*n*Pr, -*i*Pr, -*n*Bu, -*i*Bu, -*t*Bu and -CF₃), -SH, -S-CO-C₁₋₆ alkyl, -S-C₁₋₆ alkyl, -NHCOOtBu, -NHAc, phenyl, -NO₂, - C=CH, -NHCONH₂, -SO₂Me and cyclopropyl.

The substituent(s) is/are especially preferably independently selected from halogen (especially F and Cl), =O, -C₁₋₆ alkyl (e.g., -Me), -CF₃, -O-C₁₋₆ alkyl (e.g., -OMe), -OH, -NH₂, NHAc, -COOH, -CONH₂, -COCH₃ (COMe, Ac), -COO-C₁₋₆ alkyl (e.g., -COOMe), -O-C₁₋₆ alkyl (e.g., -COMe) and -NO₂.

The suffix "-ene" like e.g., in "phenylene" refers to the corresponding divalent group.

The expression alkyl refers to a saturated, straight-chain or branched hydrocarbon group that contains from 1 to 20 carbon atoms, preferably from 1 to 15 carbon atoms, especially from 1 to 10 (e.g., 1, 2, 3 or 4) carbon atoms, for example a methyl (Me, CH₃), ethyl (Et), *n*-propyl (*n*Pr), *iso*-propyl (*i*Pr), *n*-butyl (*n*Bu), *iso*-butyl (*i*Bu), *sec*-butyl (*s*Bu), *tert*-butyl (*t*Bu), *n*-pentyl, *iso*-pentyl, *n*-hexyl, 2,2-dimethylbutyl or *n*-octyl group.

Especially preferred alkyl groups are C₁₋₆ alkyl groups; moreover preferred alkyl groups are C₁₋₄ alkyl groups.

The expression C₁₋₆ alkyl refers to a saturated, straight-chain or branched hydrocarbon group that contains from 1 to 6 carbon atoms. The expression C₁₋₄ alkyl refers to a saturated, straight-chain or branched hydrocarbon group that contains from 1 to 4 carbon atoms. Examples are a methyl, ethyl, *n*-propyl, *iso*-propyl, *n*-butyl, *iso*-butyl, *sec*-butyl or *tert*-butyl group.

The expressions alkenyl and alkynyl refer to at least partially unsaturated, straight-chain or branched hydrocarbon groups that contain from 2 to 20 carbon atoms, preferably from 2 to 15 carbon atoms, especially from 2 to 10 (e.g., 2, 3 or 4) carbon atoms, for example an ethenyl (vinyl), propenyl (allyl), isopropenyl, butenyl, ethynyl (acetylenyl), propynyl (e.g., propargyl), butynyl, isoprenyl or hex-2-enyl group.

Preferably, alkenyl groups have one or two (especially preferably one) double bond(s), and alkynyl groups have one or two (especially preferably one) triple bond(s).

Furthermore, the terms alkyl, alkenyl and alkynyl refer to groups in which one or more hydrogen atoms have been replaced by a halogen atom (preferably F or CI) such as, for example, a 2,2,2-trichloroethyl or a trifluoromethyl group.

The expression heteroalkyl refers to an alkyl, alkenyl or alkynyl group in which one or more (preferably 1 to 8; especially preferably 1, 2, 3 or 4) carbon atoms have been replaced by an oxygen, nitrogen, phosphorus, boron, selenium, silicon or sulfur atom (preferably by an oxygen, sulfur or nitrogen atom) or by a SO or a SO₂ group. The expression heteroalkyl furthermore refers to a carboxylic acid or to a group derived from a carboxylic acid, such as, for example, acyl, acylalkyl, alkoxycarbonyl, acyloxy, acyloxyalkyl, carboxyalkylamide or alkoxycarbonyloxy. Furthermore, the term heteroalkyl refers to groups in which one or more hydrogen atoms have been replaced by a halogen atom (preferably F or CI).

Preferably, a heteroalkyl group contains from 1 to 12 carbon atoms and from 1 to 8 heteroatoms selected from oxygen, nitrogen and sulfur (especially oxygen and nitrogen). Especially preferably, a heteroalkyl group contains from 1 to 6 (e.g., 1, 2, 3 or 4) carbon atoms and 1, 2, 3 or 4 (especially 1, 2 or 3) heteroatoms selected from oxygen, nitrogen and sulfur (especially oxygen and nitrogen). The term C₁-C₁₀ heteroalkyl refers to a heteroalkyl group containing from 1 to 10 carbon atoms and 1, 2, 3, 4, 5 or 6 heteroatoms selected from O, S and/or N (especially O and/or N). The term C₁-C₆ heteroalkyl refers to a heteroalkyl group containing from 1 to 6 carbon atoms and 1, 2, 3 or 4 heteroatoms selected from O, S and/or N (especially O and/or N). The term C₁-C₄ heteroalkyl refers to a heteroalkyl group containing from 1 to 4 carbon atoms and 1, 2 or 3 heteroatoms selected from O, S and/or N (especially O and/or N).

Further preferably, the expression heteroalkyl refers to an alkyl group as defined above (straight-chain or branched) in which one or more (preferably 1 to 6; especially preferably 1, 2, 3 or 4) carbon atoms have been replaced by an oxygen, sulfur or nitrogen atom or a CO group or a SO group or a SO₂ group; this group preferably contains from 1 to 6 (e.g. 1, 2, 3 or 4) carbon atoms and 1, 2, 3 or 4 (especially 1, 2 or 3) heteroatoms selected from oxygen, nitrogen and sulfur (especially oxygen and nitrogen); this group may preferably be substituted by one or more (preferably 1 to 6; especially preferably 1, 2, 3 or 4) fluorine, chlorine, bromine or iodine atoms or OH, =O, SH, =S, NH₂, =NH, N₃, CN or NO₂ groups.

Examples of heteroalkyl groups are groups of formulae: R^{a}-O-Y^{a}-, R^{a}-S-Y^{a}-, R^{a}-SO-Y^{a}-, R^{a}-SO₂-Y^{a}-, R^{a}-N(R^{b})-SO₂-Y^{a}-, R^{a}-SO₂-N(R^{b})-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a bond, a C₁-C₆ alkylene, a C₂-C₆ alkenylene or a C₂-C₆ alkynylene group, wherein each heteroalkyl group contains at least one carbon atom and one or more hydrogen atoms may be replaced by fluorine or chlorine atoms.

Specific examples of heteroalkyl groups are methoxy, trifluoromethoxy, ethoxy, *n*-propyloxy, *iso*-propyloxy, *n*-butoxy, *tert*-butyloxy, methoxymethyl, ethoxymethyl, -CH₂CH₂OH, -CH₂OH, -SO₂Me, -NHAc, methoxyethyl, 1-methoxyethyl, 1-ethoxyethyl, 2-methoxyethyl or 2-ethoxyethyl, methylamino, ethylamino, propylamino, isopropylamino, dimethylamino, diethylamino, isopropylethylamino, methylamino methyl, ethylamino methyl, diisopropylamino ethyl, methylthio, ethylthio, isopropylthio, enol ether, dimethylamino methyl, dimethylamino ethyl, acetyl, propionyl, butyryloxy, acetyloxy, methoxycarbonyl, ethoxycarbonyl, propionyloxy, acetylamino or propionylamino, carboxymethyl, carboxyethyl or carboxypropyl, *N*-ethyl-*N*-methyl-carbamoyl or *N*-methylcarbamoyl. Further examples of heteroalkyl groups are nitrile (-CN), isonitrile, cyanate, thiocyanate, isocyanate, isothiocyanate and alkylnitrile groups.

The expression cycloalkyl refers to a saturated or partially unsaturated (for example, a cycloalkenyl group) cyclic group that contains one or more rings (preferably 1 or 2), and contains from 3 to 14 ring carbon atoms, preferably from 3 to 10 (especially 3, 4, 5, 6 or 7) ring carbon atoms. The expression cycloalkyl refers furthermore to groups in which one or more hydrogen atoms have been replaced by fluorine, chlorine, bromine or iodine atoms or by OH, =O, SH, =S, NH₂, =NH, N₃ or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, propellane (e.g., [1.1.1]propellane) tetraline, cyclopentylcyclohexyl, fluorocyclohexyl or cyclohex-2-enyl group. Preferably, the expression cycloalkyl refers to a saturated cyclic group that contains one or more rings (preferably 1 or 2), and contains from 3 to 14 ring carbon atoms, preferably from 3 to 10 (especially 3, 4, 5, 6 or 7) ring carbon atoms.

The expression heterocycloalkyl refers to a cycloalkyl group as defined above in which one or more (preferably 1, 2 or 3) ring carbon atoms have been replaced by an oxygen, nitrogen, silicon, selenium, phosphorus or sulfur atom (preferably by an oxygen, sulfur or nitrogen atom) or a SO group or a SO₂ group. A heterocycloalkyl group has preferably 1 or 2 ring(s) and 3 to 10 (especially 3, 4, 5, 6 or 7) ring atoms (preferably selected from C, O, N and S). The expression heterocycloalkyl refers furthermore to groups that are substituted by fluorine, chlorine, bromine or iodine atoms or by OH, =O, SH, =S, NH₂, =NH, N₃ or NO₂ groups. Examples are a piperidyl, prolinyl, imidazolidinyl, piperazinyl, morpholinyl (e.g., -N(CH₂CH₂)₂O), urotropinyl, pyrrolidinyl, tetrahydrothiophenyl, tetrahydropyranyl, tetrahydrofuryl or 2-pyrazolinyl group and also lactames, lactones, cyclic imides and cyclic anhydrides.

The expression alkylcycloalkyl refers to groups that contain both cycloalkyl and alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings and from 3 to 10 (especially 3, 4, 5, 6 or 7) ring carbon atoms, and one or two alkyl, alkenyl or alkynyl groups (especially alkyl groups) having 1 or 2 to 6 carbon atoms.

The expression heteroalkylcycloalkyl refers to alkylcycloalkyl groups as defined above in which one or more (preferably 1, 2 or 3) carbon atoms have been replaced by an oxygen, nitrogen, silicon, selenium, phosphorus or sulfur atom (preferably by an oxygen, sulfur or nitrogen atom) or a SO group or a SO₂ group. A heteroalkylcycloalkyl group preferably contains 1 or 2 rings having from 3 to 10 (especially 3, 4, 5, 6 or 7) ring atoms, and one or two alkyl, alkenyl, alkynyl or heteroalkyl groups (especially alkyl or heteroalkyl groups) having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl refers to an aromatic group that contains one or more rings and from 6 to 14 ring carbon atoms, preferably from 6 to 10 (especially 6) ring carbon atoms. The expression aryl refers furthermore to groups that are substituted by fluorine, chlorine, bromine or iodine atoms or by OH, SH, NH₂, N₃ or NO₂ groups. Examples are the phenyl (Ph), naphthyl, biphenyl, 2-fluorophenyl, anilinyl, 3-nitrophenyl or 4-hydroxyphenyl group.

The expression heteroaryl refers to an aromatic group that contains one or more rings and from 5 to 14 ring atoms, preferably from 5 to 10 (especially 5 or 6 or 9 or 10) ring atoms, comprising one or more (preferably 1, 2, 3 or 4) oxygen, nitrogen, phosphorus or sulfur ring atoms (preferably O, S or N). The expression heteroaryl refers furthermore to groups that are substituted by fluorine, chlorine, bromine or iodine atoms or by OH, SH, N₃, NH₂ or NO₂ groups. Examples are pyridyl (e.g. 4-pyridyl), imidazolyl (e.g. 2-imidazolyl), phenylpyrrolyl (e.g., 3-phenylpyrrolyl), thiazolyl, isothiazolyl, 1,2,3-triazolyl, 1,2,4-triazolyl, oxadiazolyl, thiadiazolyl, indolyl, indazolyl, tetrazolyl, pyrazinyl, pyrimidinyl, pyridazinyl, 4-hydroxypyridyl (4-pyridonyl), 3,4-hydroxypyridyl (3,4-pyridonyl), oxazolyl, isoxazolyl, triazolyl, tetrazolyl, isoxazolyl, indazolyl, indolyl, benzimidazolyl, benzoxazolyl, benzisoxazolyl, benzthiazolyl, pyridazinyl, quinolinyl, isoquinolinyl, pyrrolyl, purinyl, carbazolyl, acridinyl, pyrimidyl, 2,3'-bifuryl, pyrazolyl (e.g., 3-pyrazolyl) and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are phenylcyclopentyl, cyclohexylphenyl as well as groups derived from toluene, xylene, mesitylene, styrene, benzyl chloride, o-fluorotoluene, 1*H*-indene, tetraline, dihydronaphthalene, indanone, cumene, fluorene and indane. An aralkyl group preferably contains one or two aromatic ring systems (especially 1 or 2 rings), each containing from 6 to 10 carbon atoms and one or two alkyl, alkenyl and/or alkynyl groups containing from 1 or 2 to 6 carbon atoms and/or a cycloalkyl group containing 3, 4, 5, 6 or 7 ring carbon atoms.

The expression heteroaralkyl refers to groups containing both aryl and/or heteroaryl groups and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (especially 1 or 2 rings), each containing from 5 or 6 to 9 or 10 ring atoms (preferably selected from C, N, O and S) and one or two alkyl, alkenyl and/or alkynyl groups containing 1 or 2 to 6 carbon atoms and/or one or two heteroalkyl groups containing 1 to 6 carbon atoms and 1, 2 or 3 heteroatoms selected from O, S and N and/or one or two cycloalkyl groups each containing 3, 4, 5, 6 or 7 ring carbon atoms and/or one or two heterocycloalkyl groups, each containing 3, 4, 5, 6 or 7 ring atoms comprising 1, 2, 3 or 4 oxygen, sulfur or nitrogen atoms.

Examples are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroaryl-heterocycloalkyl, heteroarylheterocycloalkenyl, heteroarylalkylcycloalkyl, heteroaryl-alkylheterocycloalkenyl, heteroarylheteroalkylcycloalkyl, heteroarylheteroalkyl-cycloalkenyl and heteroarylheteroalkylheterocycloalkyl groups, the cyclic groups being saturated or mono-, di- or tri-unsaturated. Specific examples are a tetrahydroisoquinolinyl, benzoyl, phthalidyl, 2- or 3-ethylindolyl, 4-methylpyridino, 2-, 3- or 4-methoxyphenyl, 4-ethoxyphenyl, 2-, 3- or 4-carboxyphenylalkyl group.

As already stated above, the expressions cycloalkyl, heterocycloalkyl, alkylcycloalkyl, heteroalkylcycloalkyl, aryl, heteroaryl, aralkyl and heteroaralkyl also refer to groups that are substituted by fluorine, chlorine, bromine or iodine atoms or by OH, =O, SH, =S, NH₂, =NH, N₃ or NO₂ groups.

The term halogen refers to F, Cl, Br or I.

When an aryl, heteroaryl, cycloalkyl, alkylcycloalkyl, heteroalkylcycloalkyl, heterocycloalkyl, aralkyl or heteroaralkyl group contains more than one ring, these rings may be bonded to each other via a single or double bond or these rings may be annulated or fused or bridged.

Owing to their substitution, the compounds of the present invention may contain one or more centers of chirality. The present invention therefore includes both all pure enantiomers and all pure diastereomers and also mixtures thereof in any mixing ratio. The present invention moreover also includes all *cis*/*trans*-isomers of the compounds of the present invention and also mixtures thereof. The present invention moreover includes all tautomeric forms of the compounds of the present invention.

The present invention further provides pharmaceutical compositions comprising one or more compounds described herein or a salt (especially a pharmaceutically acceptable salt), solvate or hydrate thereof, optionally in combination with one or more carrier substances and/or one or more adjuvants. The pharmaceutical composition of the present invention may contain a further antibacterial compound.

The compounds or pharmaceutical compositions of the present invention may be administered alone (single treatment) or in combination with a further antibacterial compound; for example, in combination with chloramphenicol, gentamicin, kanamycin, neomycin, levofloxacin, moxifloxacin, tobramycin, ofloxacin, oxytetracyclin and meropenem.

The present invention provides compounds or pharmaceutical compositions as described herein for use in the treatment of *Pseudomonas* Keratitis.

The present invention especially provides compounds or pharmaceutical compositions as described herein for use in the definitive adjunctive therapy of keratitis, especially contact lens-associated keratitis (CLAK), due to confirmed *P. aeruginosa.*

Thereby, the preferred target population are patients with proven keratitis based on infection with *P. aeruginosa.*

Treatment of patients with compounds or pharmaceutical compositions as described herein results in reduced inflammation in the eye, reduced number of CFUs, reduced size and depth of stromal scar, final visual acuity.

The compounds or pharmaceutical compositions as described herein are especially useful in the treatment of patients having liquefactive necrosis of the cornea with major stromal melting and perforation in contact lens-associated keratitis (CLAK).

The present invention further provides a compound as described herein or a pharmaceutical composition as defined herein for the preparation of a medicament for the treatment of *Pseudomonas* Keratitis.

Examples of salts (especially of pharmacologically acceptable salts) of sufficiently basic compounds are salts of physiologically acceptable mineral acids like hydrochloric, hydrobromic, sulfuric and phosphoric acid; or salts of organic acids like methanesulfonic, *p*-toluenesulfonic, lactic, acetic, trifluoroacetic, citric, succinic, fumaric, maleic and salicylic acid. Further, a sufficiently acidic compound may form alkali or earth alkali metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts; all of which are also further examples of salts of the compounds described herein.

The compounds described herein may be solvated, especially hydrated. The solvation/ hydration may occur during the process of production or as a consequence of the hygroscopic nature of the initially water-free compounds. The solvates and/or hydrates may e.g. be present in solid or liquid form.

The therapeutic use of the compounds described herein, their pharmacologically acceptable salts, solvates and hydrates, respectively, as well as formulations and pharmaceutical compositions also lie within the scope of the present invention.

In general, the compounds and pharmaceutical compositions described herein will be administered by using the established and acceptable modes known in the art. Topical administration is preferred.

According to a moreover preferred embodiment, the present invention provides a method for treatment of *Pseudomonas* Keratitis, which comprises administering to a subject in need of such treatment a therapeutically effective amount of a compound described herein, or a salt (especially a pharmaceutically acceptable salt) thereof.

According to a further preferred embodiment, the present invention provides a method for treatment of *Pseudomonas* Keratitis, which comprises administering to a subject in need of such treatment a pharmaceutical composition comprising a compound described herein, or a salt (especially a pharmaceutically acceptable salt) thereof.

The compounds disclosed herein can be synthesized as e.g. described in WO 2023/166039 A1 and WO 2022/043322 A1.

### EXAMPLES

### Synthetic procedure for HIPS-7178:

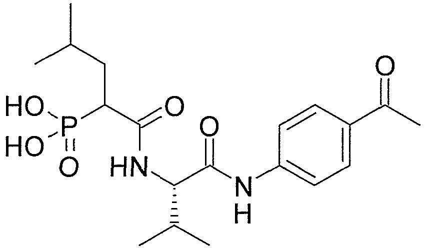

(2-(diethoxyphosphoryl)-4-methylpentanoyl)-L-valine (114 mg, 0.32 mmol) was reacted with 4-aminoacetophenone (47 mg, 0.35 mmol), NMM (89 µL, 0.80 mmol) and TBTU (112 mg, 0.35 mmol) in DCM (3.2 mL) to afford (1-(((S)-1-((4-Methoxyphenyl)amino)-3-methyl-1-oxobutan-2-yl)amino)-4-methyl-1-oxopentan-2-yl)phosphonic acid (139 mg, 0.30 mmol, 94% yield, which was taken to the next step without further purification. (1-(((S)-1-((4-Methoxyphenyl)amino)-3-methyl-1-oxobutan-2-yl)amino)-4-methyl-1-oxopentan-2-yl)phosphonic acid (139 mg, 0.30 mmol) was deprotected by bromotrimethylsilane (0.28 mL, 2.1 mmol) in DCM (3.0 mL). Then, MeOH was added and stirred at room temperature for 30 min to cleave the previously formed TMS ester. The solvents were removed under reduced pressure and the crude product was purified *via* a Dionex Ultimate 3000 (Thermo Scientific) with a Nucleodur C18 Graphity column (250mm × 16 mm, particle size 5µm) using UV-detection to afford the title compound (34 mg, 0.082 mmol, 27% yield) as a pale-pink solid.

**Animals.** The animal experiments were approved by Saarland University's Animal Welfare Committee (Application number: 17/2021) and conducted in accordance with German and European recommendations and guidelines for the ethical use of animals. The laboratory animals were all C57BL/6N female eight to eleven months old mice. Unlimited access to water and show was secured. Temperature was closely maintained around 22 °C and relative air humidity around 50%. Light and darkness alternated at twelve-hour intervals. All laboratory animals were purchased from Charles River GmbH, Sulzfeld, Germany.

***Pseudomonas aeruginosa.*** The *P. aeruginosa* strain PA54 was employed to induce an experimental bacterial keratitis in the aforementioned C57BL/6N mice. The strain PA54 was isolated from a human infection (wound swab from the eye) and collected at Saarland University Medical Center's Institute for Medical Microbiology and Hygienics (IMMH) in 2009. According to Magiorakos *et al.,* PA54 can be classified as multidrug-resistant due to its lacking sensibility to the anti-pseudomonal cephalosporins ceftazidime and cefepime, the anti-pseudomonal fluoroquinolones ciprofloxacin and levofloxacin, the phosphoric acid fosfomycin, and the aminoglycoside gentamicin. The minimum inhibitory concentration (MIC) of the reserve antibiotic meropenem (meropenem trihydrate, Sigma) was 1 µg/mL (sensitivity limit = 2 µg/mL). The *exoU-* and exoT-positivity of the PA54 strain, foregrounds its keratitogenic potential.

**Inoculum.** The *P. aeruginosa* strain PA54 is routinely grown overnight on blood agar (TrypticaseTM Soy Agar, 5% sheep blood, BD, Heidelberg, Germany) at 37 °C. To obtain a stationary phase cell inoculum, a freshly grown colony was inoculated in tryptic soy broth (TSB; BD, Heidelberg, Germany) and cultured at 37 °C and 150 rounds per minute (rpm) for 16 h. Then, 2 mL were centrifuged at 13,200 rpm at rt for 2 min, whereupon cells were resuspended in spent medium to an OD₆₀₀ of 10 (~1×10¹⁰ CFU/mL). Short storage on ice followed, before the infection procedure started.

**Infection procedure.** Anesthesia and infection were performed as previously published. Briefly, fentanyl (Hameln Pharma Plus GmbH, Hameln, Germany), midazolam hydrochloride (Midazolam-Hameln, 5 mg/mL, Hameln pharma GmbH, Germany) and medetomidine hydrochloride (Domitor, Orion Corporation, Espoo, Finland) were dissolved in 0.9% NaCl (NaCl 0.9%, Ecotainer 500 mL, B. Braun, Melsungen, Germany), dosed at 0.05 mg/kg, 5 mg/kg and 0.5 mg/kg body weight, respectively, and applied by intraperitoneal injection. Analgesia (Carprofen, 5 mg/kg body weight, Zoetis Deutschland GmbH, Berlin, Germany) was applied subcutaneously. Lacrimation was stopped with 10 µL of a 0.6% acetylcysteine solution (Pharmacy of Saarland University Hospitals, Homburg, Germany). Local analgesia (0.5% proxymetacaine hydrochloride, Proparakain-POS Augentropfen, Ursapharm Arzneimittel GmbH, Saarbrücken, Germany) was eye-dropped and removed after 60 seconds. The blunt side of a scalpel was used to pass five times over the cornea to remove the superficial epithelium. Then, three parallel, vertical, 1-2 mm long scratches were added to the cornea with a PA54-loaded 27-Gauge needle (B. Braun). Afterwards, 5 µL (~5×10⁷ CFU/mL) of the inoculum were pipetted onto the scratched eye and left untouched for 20 min (Preston et al., 1995; Wu et al., 2017). Finally, 1.2 mg/kg body weight naloxone hydrochloride (Naloxon Inresa, 0.4 mg/mL, Inresa Arzneimittel GmbH, Freiburg, Germany), 0.5 mg/kg flumazenil (Flumazenil Inresa 0.5 mg, 0.1 mg/mL, Inresa Arzneimittel GmbH) and 2.5 mg/kg atipamezole hydrochloride (Antisedan, 5 mg/ml, Vetoquinol GmbH, Ismaning, Germany) were dissolved in 0.9% NaCl and injected subcutaneously, awakening the mouse.

**Documentation and Treatment.** Documentation was performed every 24 h. Keratitis severity was visualized using a stereomicroscope (Leitz ELVAR, Wetzlar, Germany) at 16× magnification combined with a high-resolution microscope digital camera (Seben GmbH, Berlin, Germany) and evaluated using a previously described clinical scoring scheme (Lyu et al., 2020). Briefly, a score of 0 indicates no infiltrates, 1 indicates that less than 50% of the cornea is semitransparent, 2 indicates that more than 50% of the cornea is semitransparent, 3 indicates that less than 50% of the cornea is opaque, 4 indicates that more than 50% of the cornea is opaque, and 5 indicates that either spontaneous corneal perforation or phthisis bulbi has occurred. The body weight of the mice was measured daily and used as welfare indicator. General condition and behavior were also recorded daily. Treatment was performed every eight hours starting six hours after infection along 72h. Anesthesia was reached using 3% isoflurane inhalation (Isoflurane-Piramal, Piramal Critical Care Deutschland GmbH, Halbergmoss, Germany) under stable oxygen supply (1.0-1.2 L/min). Then a volume of 5 µL of the appropriate substance was applied to the infected eye. A two-minute incubation period followed. The animal was finally reawakened by isoflurane-removal. Four treatment groups were created. The sham treatment group was applied phosphate buffered saline containing 1% DMSO. The meropenem treatment group was applied a 0.9% NaCl solution containing 500 µg meropenem/mL and 1% DMSO. The LasB-inhibitor treatment group was applied a 0.9% NaCl solution containing 1 mg LasB-inhibitor/mL and 1% DMSO. The combination treatment group was applied a 0.9% NaCl solution containing 1 mg LasB-inhibitor/mL, 500 µg meropenem/mL, and 1% DMSO.

**Animal Sacrifice.** Laboratory animals were sacrificed by intraperitoneal injection of ketamine hydrochloride/xylazine hydrochloride (Ursotamine, 100 mg/mL, Serumwerk Bernburg AG, Germany; Rompun 2%, xylazine, 25 ml, Bayer AG) at a dose of 20 mg/kg body weight three days after infection. The vena cava served was punctured to collect blood. Anticoagulation was achieved by Ca²⁺-deprivation using a drop of 0.5 M ethylenediaminetetraacetic acid (EDTA). The right and initially infected eye was removed by incising the surrounding skin if necessary.

**Eye Homogenization.** The removed eyes were homogenized using a POLYTRON PT 1200 E dispenser (Kinematika AG, Lucerne, Switzerland). To avoid bacterial carry-over, the eyes were cleaned with distilled water, 70% ethanol and 0.9% NaCl solution, respectively.

**Microbiological Evaluation.** Serial dilutions were plated on blood agar for microbiological evaluation of the homogenates. A blood stripe also incubated on blood agar served as a control for potential systemic spread of the pathogen. In both cases, counting was performed after 18h of incubation at 37°C.

**Flow Cytometry.** The eye homogenates, upon receipt, were sieved using a 70 µm cell filter (Falcon, Corning) to prepare a single cell suspension. Total cell count and cell viability were determined using a NucleoCounter^{®} NC-200^{™} (Chemomatec, Kaiserslautern, Germany). Single cell suspensions were fixed using 1% paraformaldehyde, centrifuged, and resuspended in FACS buffer (0.1mM EDTA plus 1% fetal bovine serum in PBS). CD16/CD32 antibodies (Thermo Fisher Scientific) were then added to the samples and incubated at 4 °C for 40 min to block Fc receptors. The surface markers were stained using the following fluorescent dye-conjugated antibodies: F4/80-PE (clone BM8), CD45- APC (clone 30-F11), CD11b-FITC (clone M1/70), CD11cAPC-Cy7 (clone N418), Ly6G PE-Cy7 (1A8-Ly6g), and NK1.1-PerCP-Cy5.5 (clone PK136) (Biolegend) and incubated at 4 °C for 45 min. The blood samples were first mixed with an ACK-Lysis Buffer (Thermo Fisher Scientific) for 20 min. The lysis was terminated in time by adding DPBS. Centrifugation was used to selectively collect the leukocytes. Total cell count estimation and surface marker staining were performed as described above for the eye samples. The BD FACSverse machine was used for flow cytometry analysis, whereas the resulting data were analyzed using Flowjo v10.6.2 (BD).

**Enzyme-linked Immunoadsorption Assay.** After centrifugation of the homogenates at 2500 g and 4 °C for 10 min, the cell-free suspension was frozen at -70 °C. Quantification of mKC, mTNF-α, mMPO and mIL-1β in the centrifuged homogenate was performed using R&D ELISA kits according to the manufacturer's protocol. Briefly, 96-well microplates were coated with the respective capture antibody and incubated at rt overnight. The following day, the wells were blocked with Delent reagent for 2h, incubated with the samples for 2h, incubated with the capture antibody for 2h and finally incubated with streptavidin-HRP for 20 min. The reaction was started by adding the substrate solution and stopped after 20 min with 2NH2SO4. The absorbance was measured at 450 nm. A serially diluted standard was used in parallel to calculate the concentration of each protein.

**Bioanalytical sample preparation of keratitis samples for determination of concentration of HIPS-7178 in eye and serum.** All PD serum and eye homogenate samples were analyzed via HPLC-MS/MS using an Agilent 1290 Infinity II HPLC system and coupled to an AB Sciex QTrap6500+ mass spectrometer. First, a calibration curve was prepared by spiking different concentrations of **HIPS-7178** into the respective matrix (mouse serum (pooled, from CD-1 mice) for plasma samples, isotonic sodium chloride solution for eye homogenate samples). Caffeine was used as an internal standard. In addition, quality control samples (QCs) were prepared for **HIPS-7178** with the respective matrix. The following extraction procedure was used: 7.5 µL of a serum sample (calibration samples, QCs or PD samples) was extracted with 37.5 µL of methanol containing 12.5 ng/mL of caffeine as internal standard for 5 min at 2,000 rpm on an Eppendorf MixMate^{®} vortex mixer. Then samples (serum) were spun down at 13,000 rpm for 5 min. Supernatants were transferred to standard HPLC-glass vials. 50 µL of an eye homogenate sample (calibration samples, QCs or PD samples) were extracted with 50 µL of methanol and 1 µL caffeine (concentration 1 µg/mL in methanol) for 5 min at 800 rpm on an Eppendorf MixMate^{®} vortex mixer. Then samples (eye homogenate) were spun down at 4,000 rpm for 40 min at 4 °C. Supernatants were transferred to 96well V-bottom plates (Greiner). HPLC conditions were as follows: column: Agilent Zorbax Eclipse Plus C18, 50x2.1 mm, 1.8 µm; temperature: 30 °C; injection volume: 5 µL; flow rate: 700 µL/min; solvent A: water + 0.1% formic acid; solvent B: acetonitrile + 0.1% formic acid; gradient: 99% A at 0 min and until 1 min, 99% - 0% A from 1.0 min to 2.2 min, 0% A until 4 min. Mass spectrometric conditions were as follows: Scan type: MRM, negative and positive mode; Q1 and Q3 masses for caffeine and **HIPS-7178** can be found in table given below. Peak areas of each sample and of the corresponding internal standard were analyzed using MultiQuant 3.0 software (AB Sciex). Peak areas of the respective sample were normalized to the internal standard peak area. The MS/MS pairs used for quantification are marked with a 'Q' in the table, the other MS/MS pairs for the respective compound were used for qualification. Peaks of PD samples were quantified using the calibration curve. The accuracy of the calibration curve was determined using QCs independently prepared on different days.

Q1 and Q3 masses for caffeine and **HIPS-7178** (MS/MS pairs used for quantification are marked with a 'Q')

| **ID** | **Q1 Mass [Da]** | **Q3 Mass [Da]** | **time [msec]** | **DP [volts]** | **CE [volts]** | **CXP [volts]** |
|---|---|---|---|---|---|---|
| **Caffeine** | 195.024 | 138.000 (Q) | 30.0 | 130.0 | 25.0 | 14.0 |
| | | 110.000 | 30.0 | 130.0 | 31.0 | 18.0 |
| **HIPS-7178** | 411.125 | 78.8 (Q) | 30.0 | -95.0 | -82.0 | -35.0 |
| | | 294.9 | 30.0 | -95.0 | -40.0 | -13.0 |

**LC-MS/MS assay for determination of LasB and desmosin in eye homogenate samples.** All samples were analyzed via HPLC-MS/MS using an Agilent 1290 Infinity II HPLC system and coupled to an AB Sciex QTrap7500 mass spectrometer. Eye homogenate samples from the PD study with **HIPS-7178** were extracted as follows: 15 µL of an eye homogenate sample was treated with the Sciex Protein Preparation Kit (Darmstadt, Germany, AB Sciex). In brief, 30 µl of digestion buffer (0.1 M Tris, pH 8, 4 mM CaCl₂), then 2.5 µl Denaturant (10 % N-octyl-glucoside) and 5 µl reducing agent (50 mM of tris (2-caroxyethyl)-phosphine were added to each sample. Then the mixture was incubated for 1 hour at 60°C at 150 rpm on a rotary shaker. Then samples were shortly spun down. Next, 2.5 µl cysteine blocking reagent (200 mM methyl methane-thiosulfanate) were added and the mixture was incubated for 10 min at room temperature. Then, 50 µl of digestion buffer was added and finally 10 µl of trypsin (/dissolved in 0.1 % formic acid). The mixture was incubated for 3 hours at 37°C. Then samples were quickly spun down and reaction was terminated with 5 µl of a quenching solution containing 10% formic acid in water. Then samples were spun down at 4,000 rpm at room temperature for 10 min. Supernatants were transferred to new 96well-Greiner-V-bottom plates. HPLC conditions were as follows: column: Agilent Zorbax Eclipse Plus C18, 50x2.1 mm, 1.8 µm; temperature: 30 °C; injection volume: 5 µL; flow rate: 700 µL/min; solvent A: water + 0.1% formic acid; solvent B: acetonitrile + 0.1% formic acid; gradient: 99% A at 0 min and until 1 min, 99% - 0% A from 1.0 min to 3 min, 0% A until 4.7 min. Mass spectrometric conditions were as follows: Scan type: MRM, positive mode; Source temperature: 500°C; Spray voltage: 2000 V; Ion source gas1: 40, Ion source gas2: 70. Q1 and Q3 masses for LasB can be found in the litereature (doi: 10.1021/acscentsci.3c01102).

**Statistical Analysis.** Statistical analysis was performed using the GraphPad Prism Software Package (Version 10.0.2). Pairwise comparison for connected and not-connected samples was done with the non-parametric Mann-Whitney U test and with the Wilcoxon-signed-rank-test respectively. The non-parametric Friedman test was employed for multiple comparisons for connected samples. The P-value was two-sided and considered statistically significant when < 0.05.

**Figure 1** shows impact of drug treatment on keratitis development in C57BL/6N mice after infection with the PA strain PA54. **A:** Evolution of clinical score from day 1 to day 3. **B:** Determination of bacterial loads in whole-eye homogenates at 3 days post infection. Data represent the values of every individual clinical score/CFU rate per animal (symbols; *n* = 8 per group) and the median (horizontal line). **, *P*<0.01 (Mann-Whitney *U* test between 2 groups). LasB-inhibitor in this experiment: **HIPS-7178.** Combination: **HIPS-7178** plus Meropenem.

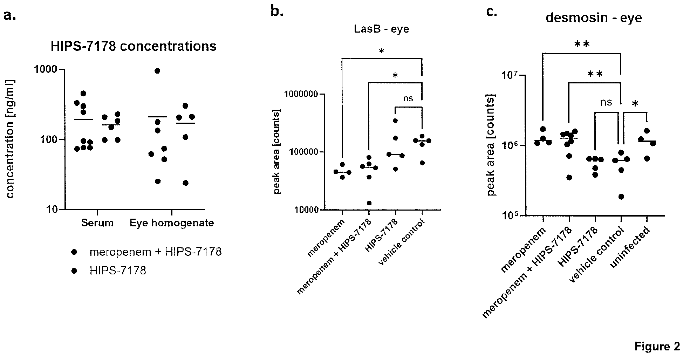

**Figure 2** shows **HIPS-7178,** LasB and desmosin levels in the keratitis model. (a) **HIPS-7178** concentrations in eye homogenate and serum were similarly high and no difference were observed between combination and **HIPS-7178** only group. (b). LasB levels were determined using LC-MS/MS in eye homogenate. Vehicle showed highest levels, whereas **HIPS-7178,** meropenem and combination group reduced levels. *: p < 0.05. (c) Desmosin levels in eye were determined for meropenem, combination group, **HIPS-7178,** vehicle control and uninfected control. Meropenem and combination group showed similarly high levels as uninfected group.

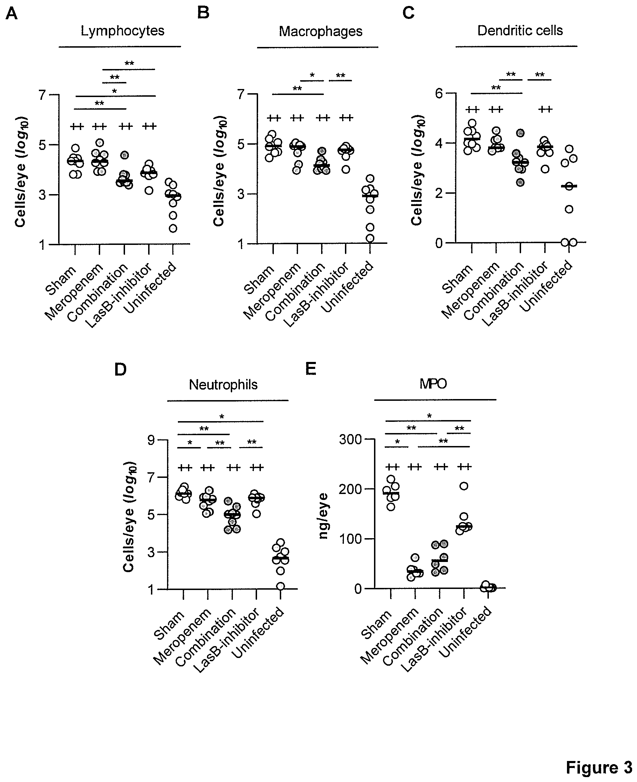

**Figure 3** shows impact of drug treatment on the immune cell content in PA54-infected eyes. **A-D:** Determination of the ocular total lymphocyte (A), macrophage (B), dendritic cell (C), and neutrophil (D) numbers at 3 days post infection (n = 7 and 8 for uninfected and infected groups, respectively). E: Myeloperoxidase (MPO) contents in whole eye homogenates obtained from eyes at 3 days post infection (*n* = 6 per group). Data represent the values of every individual animal (symbols) and the median (horizontal line). +, *P* < 0.05; ++, *P* < 0.01 (Mann-Whitney *U* test between uninfected group and infected groups). *, *P* < 0.05; **, *P* < 0.01 (Mann-Whitney *U* test between 2 infected groups). LasB-inhibitor in this experiment: **HIPS-7178.** Combination: **HIPS-7178** plus Meropenem.

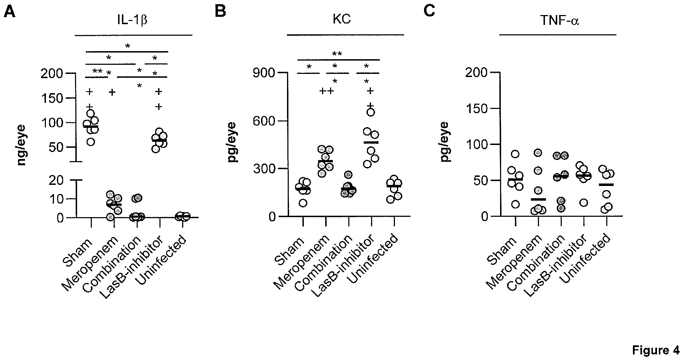

**Figure 4** shows impact of drug treatment on selected inflammatory markers in PA54-infected eyes at 3 days post infection. A-C: Interleukin 1β (IL-1β; A), keratinocyte chemoattractant (KC; B), and tumor necrosis factor-α (TNF-α; C) loads per eye (n = 6 per group). Single points represent the values of each eye cell count per animal (symbols). Data represent the values of every individual animal (symbols) and the median (horizontal line). +, P < 0.05; ++, P < 0.01 (Mann-Whitney U test between uninfected group and infected groups). *, P < 0.05; **, P < 0.01 (Mann-Whitney U test between 2 infected groups). LasB-inhibitor in this experiment: HIPS-7178. Combination: HIPS-7178 plus Meropenem.

## Claims

1. A compound of formula (I): 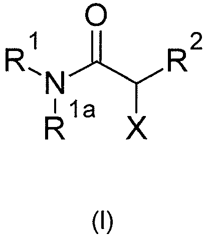 wherein
X is a group of formula -PO(OH)₂, -SH, -C(=O)-NH-OH, an optionally substituted triazolyl group, -SR³, -PO(OH)(OR⁴) or -PO(OR⁴)(OR⁵);
R¹ is an optionally substituted cycloalkyl group, an optionally substituted heterocycloalkyl group, an optionally substituted aryl group or an optionally substituted heteroaryl group or an optionally substituted aralkyl group or an optionally substituted heteroaralkyl group; or a group of formula -CH(R⁶)-C(=O)-NH-R⁷, or a group of formula -C(Me)₂-CH₂-C(=O)-NH-R⁷, or a group of formula -CH(R⁶)-CH₂-C(=O)-NH-R⁷, or a group of formula -CH(R⁶)-R⁸;
R² is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R³ is a group of formula -COR^{3a} or -CON(R^{3b})₂; wherein R^{3a} is an alkyl group, an optionally substituted phenyl group or an optionally substituted benzyl group and R^{3b} is independently selected from hydrogen or an alkyl group, an optionally substituted phenyl group or an optionally substituted benzyl group;
R⁴ is an alkyl group, an optionally substituted phenyl group or an optionally substituted benzyl group;
R⁵ is an alkyl group, an optionally substituted phenyl group or an optionally substituted benzyl group;
R⁶ is hydrogen or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
R⁷ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group;
R⁸ is an optionally substituted cycloalkyl group, an optionally substituted heterocycloalkyl group, an optionally substituted aryl group, an optionally substituted heteroaryl group, an optionally substituted aralkyl group or an optionally substituted heteroaralkyl group; and
R^{1a} is hydrogen, or, if R¹ is a group of formula -CH(R⁶)-C(=O)-NH-R⁷, R^{1a} and R⁶ together may be a group of formula -(CH₂)₃- or -(CH₂)₄-;
or a salt thereof,
for use in the treatment of *Pseudomonas* Keratitis.

2. The compound for use according to claim 1, wherein the compound is a compound of formula (III): 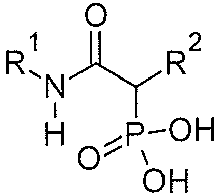 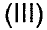 wherein R¹ and R² are as defined in claim 1, or a salt thereof.

3. The compound for use according to claim 1 or 2, wherein R² is a C₁₋₆ alkyl group;
a heteroalkyl group containing from 1 to 6 carbon atoms and 1, 2, 3 or 4 heteroatoms selected from O, S and N; a C₄₋₁₀ alkylcycloalkyl group; or a C₇₋₁₂ aralkyl group; all of which may optionally be substituted;
especially wherein R² is an optionally substituted benzyl group, or a group of formula -CH₂CH(CH₃)₂;
further especially wherein R² is a group of formula -CH₂CH(CH₃)₂.

4. The compound for use according to any one of claims 1 to 3, wherein R¹ is an optionally substituted aryl group or an optionally substituted heteroaryl group;
especially wherein R¹ is an optionally substituted phenyl group, an optionally substituted naphthyl group or an optionally substituted heteroaryl group containing one or two rings and from 5 to 10 ring atoms selected from C, O, N and S;
further especially wherein R¹ is an optionally substituted phenyl group.

5. The compound for use according to any one of claims 1 to 3, wherein R¹ is a group of formula -Cy¹-L-Cy², wherein Cy¹ is an optionally substituted cycloalkylene group containing 1 or 2 rings and from 3 to 7 carbon ring atoms, an optionally substituted heterocycloalkylene group containing 1 or 2 rings and from 3 to 7 ring atoms selected from C, N, O and S, an optionally substituted phenylene group, or an optionally substituted heteroarylene group containing 5 or 6 ring atoms selected from C, N, O and S; Cy² is a cycloalkyl, heterocycloalkyl, alkylcycloalkyl, heteroalkylcycloalkyl, aryl, heteroaryl, aralkyl or heteroaralkyl group, all of which may optionally be substituted; and L is a bond or-O-, -S-, -NH-, -CH₂-, -CO-, -NHCO-, -CO-NH-, -CH₂-CO-NH-, -NH-CO-CH₂-, -CH₂-O-CO-NH-, -NH-CO-O-CH₂-, -O-CO-NH-, -NH-CO-O-, -NHSO₂-, - SO₂NH-, -CH₂-SO₂-NH-, -NH-SO₂-CH₂-, -S-CH₂-, -CH₂-S-, -NH-CH₂-, -CH₂-NH-, -O-CH₂- or -CH₂-O-;
especially wherein Cy² is an optionally substituted phenyl group, an optionally substituted biphenyl group, an optionally substituted naphthyl group, an optionally substituted heteroaryl group containing one or two rings and 5, 6, 9 or 10 ring atoms selected from C, O, N and S, an optionally substituted cycloalkyl group containing from 3 to 7 ring atoms, an optionally substituted heterocycloalkyl group containing from 3 to 7 ring atoms selected from C, N, O and S, an optionally substituted heterocycloalkylaryl group containing 9 or 10 ring atoms selected from C, N, S and O, or a group of formula -CH(CH₂Ph)Ph; and/or wherein L is a bond or -NHCO-, -CO-NH-, -CH₂-CO-NH-, -NH-CO-CH₂-, -NHSO₂- or -SO₂NH-; and/or wherein Cy¹ is a 1,4-phenylene group.

6. The compound for use according to any one of claims 1 to 3, wherein R¹ is a group of formula -CH(R⁶)-C(=O)-NH-R⁷ or a group of formula -CH(R⁶)-R⁸.

7. The compound for use according to claim 6, wherein R⁶ is hydrogen or a C₁₋₆ alkyl group, a C₃₋₇ cycloalkyl group, a heterocycloalkyl group containing from 3 to 7 ring atoms selected from C, N, O and S, a phenyl group or a heteroaryl group containing 5 or 6 ring atoms selected from C, N, S and O, or a group of formula -CH₂-R^{6a}, wherein R^{6a} is a C₃₋₇ cycloalkyl group, a heterocycloalkyl group containing from 3 to 7 ring atoms selected from C, N, O and S, a phenyl group or a heteroaryl group containing 5 or 6 ring atoms selected from C, N, S and O;
especially wherein R⁶ is a group of formula -CH(CH₃)₂.

8. The compound for use according to claim 6 or 7, wherein R⁷ is an optionally substituted phenyl group or an optionally substituted C₃₋₇ cycloalkyl group; and/or wherein R⁸ is an optionally substituted heteroaryl group containing 5 to 10 ring atoms selected from C, N, O and S (such as an optionally substituted benzimidazole group or an optionally substituted triazole group or an optionally substituted imidazole group).

9. A compound of formula (VII): 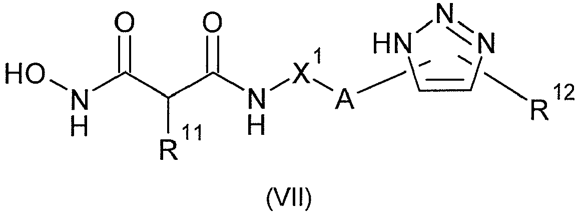 wherein
A is a bond, CH₂ or C=O (especially CH₂);
X¹ is an optionally substituted cycloalkylene group, an optionally substituted heterocycloalkylene group, an optionally substituted arylene group or an optionally substituted heteroarylene group;
R¹¹ is an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted; and
R¹² is hydrogen or an alkyl, alkenyl, alkynyl, heteroalkyl, cycloalkyl, heterocycloalkyl, alkylcycloalkyl, heteroalkylcycloalkyl, aryl, heteroaryl, aralkyl or heteroaralkyl group, all of which may optionally be substituted;
or a salt thereof,
for use in the treatment of *Pseudomonas* Keratitis.

10. A compound for use according to claim 9, wherein X¹ is an optionally substituted arylene group or an optionally substituted heteroarylene group;
especially wherein X¹ is an optionally substituted phenylene group or an optionally substituted heteroarylene group having 5 or 6 ring atoms that are selected from C, N, O and S;
further especially wherein X¹ is a 1,3 phenylene group or a 1,4 phenylene group.

11. A compound for use according to claim 9 or 10, wherein R¹¹ is a C₁₋₆ alkyl group; a heteroalkyl group containing from 1 to 6 carbon atoms and 1, 2, 3 or 4 heteroatoms selected from O, S and N; a C₃₋₇ cycloalklyl group; a C₄₋₁₀ alkylcycloalkyl group; or a C₇₋₁₂ aralkyl group; all of which may optionally be substituted;
especially wherein R¹¹ is a C₁₋₆ alkyl group; a heteroalkyl group containing from 1 to 6 carbon atoms and 1, 2, 3 or 4 heteroatoms selected from O, S and N; a C₃₋₇ cycloalklyl group; or a group of formula -CH₂-R^{11a}, wherein R^{11a} is a C₃₋₇ cycloalkyl group or an optionally substituted phenyl group;
further especially wherein R¹¹ is a C₁₋₆ alkyl group; or a group of formula -CH₂-R^{11a}, wherein R^{11a} is a phenyl group or a cyclopropyl group;
moreover especially wherein R¹¹ is a group of formula -CH₂CH(CH₃)₂ or a group of formula -CH(CH₃)₂.

12. A compound for use according to any one of the preceding claims 9 to 11, wherein R¹² is a group of formula -CH₂-NH-SO₂-R¹³ or -CH₂-N(CH₃)-SO₂-R¹³, wherein R¹³ is an optionally substituted phenyl group, an optionally substituted benzyl group, an optionally substituted C₃₋₁₀ cycloalkyl group, an optionally substituted -CH₂-C₃₋₁₀ cycloalkyl group, a C₁₋₆ alkyl group or a C₁₋₆ heteroalkyl group; or
wherein R¹² is a group of formula -CH₂-Y¹-R¹⁴, wherein Y¹ is selected from a bond, O, NH, S and NHCO; and R¹⁴ is hydrogen, a C₁₋₆ heteroalkyl group or an optionally substituted phenyl group; or
wherein R¹² is an optionally substituted phenyl group; or
wherein R¹² is selected from the following groups:

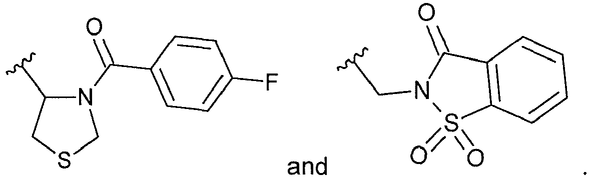

13. The compound for use according to any one of the preceding claims, for use in the definitive adjunctive therapy of keratitis, especially contact lens-associated keratitis (CLAK), due to confirmed *P*. *aeruginosa.*

14. The compound for use according to any one of the preceding claims, which is administered in combination with an antibiotic like chloramphenicol, gentamicin, kanamycin, neomycin, levofloxacin, moxifloxacin, tobramycin, ofloxacin, oxytetracyclin and meropenem.

15. A compound having the following formula, or a salt thereof:
